# EUROPÄISCHE PATENTANMELDUNG

(11) **EP 1 632 200 A1**
(43) Veröffentlichungstag der Anmeldung: **08.03.2006**
(21) Anmeldenummer: 05016868.1
(22) Anmeldetag: 03.08.2005
(51) Int. Cl.: A61F 2/42

(54) **Daumensattelgelenkprothese**

(30) Priorität: 02.09.2004 DE 102004043006
(71) Anmelder: ESKA Implants GmbH & Co., 23556 Lübeck (DE)
(72) Erfinder: Grundei, Hans, Dr.-Ing., 23558 Lübeck (DE); Aschoff, Horst-Heinrich, Dr. med., 23558 Lübeck (DE)
(74) Vertreter: Fuchs Mehler Weiss & Fritzsche

(57) **Zusammenfassung**

Es wird ein Daumensattelgelenk beschrieben, welches aufweist:
- ein in das Trapezium (12) implantierbares erstes Stielteil (1),
- ein in den Os metacarpale 1 (13) implantierbares zweites Stielteil (2),
- ein mit dem zweiten Stielteil (2) lösbar verbundenes Aufnahmeteil (3) mit einer kugelkalottenförmigen Ausnehmung (4), und
- eine mit dem ersten Stielteil (1) lösbar verbundene Gelenkkugel (5), die in der Ausnehmung (4) des Aufnahmeteils (3) liegt und bei Beugung des Gelenkes darin abrollt,
- wobei die Basiskante (11) der Ausnehmung (4) über den Äquator der Gelenkkugel (5) greift.

## Beschreibung

Die Erfindung betrifft ein Daumensattelgelenk. Es ist dazu vorgesehen, zwischen dem Trapezium und dem Os metacarpale I implantiert zu werden nach Resektion des natürlichen Gelenkes.

Künstliche Fingergelenke sind bekannt. So wird beispielsweise hingewiesen auf das in der DE 195 12 854 C beschriebene Fingergelenk. Dieses weist einen ersten und einen zweiten Verankerungsstiel auf, die in die Fingerröhrenknochen setzbar sind. Zwischen den Verankerungsstielen ist ein Scharniergelenk in Form eines Kugelgelenks angeordnet. Der erste Verankerungsstiel ist mit einem Kugelkäfig des Schamiergelenks verbunden, in welchem eine mit einem Stiel versehene Gelenkkugel gelagert ist, wobei der Stiel den Kugelkäfig durch einen darin vorgesehenen Schlitz durchtritt und mit dem zweiten Verankerungsstiel in Verbindung steht. Der Schlitz im Kugelkäfig weitet sich von der Stelle, an der der Stiel den Kugelkäfig in Streckstellung des Gelenkes durchtritt, zu der Stelle, an der der Stiel den Kugelkäfig in Beugestellung des Gelenkes durchtritt, stetig weiter auf.

Mit diesem Fingergelenk ist das Ziel verfolgt worden, ein Fingergelenk zu schaffen, welches eine hohe Genauigkeit der Nachbildung der natürlichen physiologischen Bewegung eines natürlichen Fingergelenks erreicht. Hierfür ist im Wesentlichen der sich stetig aufweitende Schlitz verantwortlich.

Das bekannte Gelenk kann als Fingergrundgelenk oder aber zwischen Fingerknöcheln implantiert werden. Für eine Implantation als Daumensattelgelenk eignet sich das bekannte Gelenk nicht. Darüber hinaus ist es aufgrund der relativen Abgeschlossenheit schwierig, im Falle eines fortgeschrittenen Abriebs der Gelenkkomponenten die Funktion unkompliziert wieder herzustellen, d.h. die verschlissenen Komponenten zu ersetzen.

Vor diesem Hintergrund ist die Aufgabe der vorliegenden Erfindung, hier Abhilfe zu schaffen. Es soll also ein Daumensattelgelenk angegeben werden, dessen verschlissene Teile in einfacher Weise ersetzt werden können, ohne dass die Verankerung des Implantates im knöchernen Material angegriffen würde.

Gelöst wird diese Aufgabe durch das erfindungsgemäße Daumensattelgelenk, welches aufweist
- ein in das Trapezium (12) implantierbares erstes Stielteil (1),
- ein in den Os metacarpale 1 (13) implantierbares zweites Stielteil (2),
- ein mit dem zweiten Stielteil (2) lösbar verbundenes Aufnahmeteil (3) mit einer kugelkalottenförmigen Ausnehmung (4), und
- eine mit dem ersten Stielteil (1) lösbar verbundene Gelenkkugel (5), die in der Ausnehmung (4) des Aufnahmeteils (3) liegt und bei Beugung des Gelenkes darin abrollt,
- wobei die Basiskante (11) der Ausnehmung (4) über den Äquator der Gelenkkugel (5) greift.

Das erfindungsgemäße Daumensattelgelenk ist im Prinzip aufgebaut wie ein künstliches Hüftgelenk in kinematischer Umkehr. Vergleicht man das Trapezium mit dem natürlichen Becken so besteht die kinematische Umkehr darin, dass mit dem Trapezium die Gelenkkugel verbunden ist, die in eine Ausnehmung im Aufnahmeteil greift, welches mit dem zweiten Stielteil lösbar verbunden ist. Das zweite Stielteil würde korrespondieren mit dem Hüftstiel bei einem künstlichen Hüftgelenk. Diese Maßnahme ist aus Platzgründen gewählt und ermöglicht einen größeren Schwenkbereich als wenn die Gelenkkugel mit dem zweiten Stielteil verbunden wäre, die dann in eine Ausnehmung im ersten Stielteil greifen würde.

Luxationsneigungen werden dadurch unterbunden, dass die Basiskante der Ausnehmung über den Äquator der Gelenkkugel greift. Das Aufnahmeteil besteht vorzugsweise aus Polyethylen, wohingegen die Gelenkkugel vorzugsweise aus einem Metall besteht. Die Gelenkteile werden dadurch unter Ausübung einer antiluxatorischen Wirkung zusammengefügt, indem die Metallkugel die Ausnehmung im Aufnahmeteil kurzzeitig spreizt, wonach dann, nachdem die Basiskante über den Äquator der Kugel geschnappt ist, diese aufgrund der Rückstellkräfte wieder in die Ausgangslage zurückfedert und so die Kugel sicher einschließt.

Bevorzugt sind die Stielteile als Hohlstiele mit gitterstrukturierter Außenwandung ausgebildet. Dies bedeutet, dass die Stielteile bildlich gesprochen aus einem zu einem Rohr geformten offenen maschenförmigen Netzwerk gebildet sind. Diese Ausbildung gestattet nicht nur ein Einwachsen von Knochentrapekeln, sondern ein regelrechtes Durchwachsen der Oberfläche des Implantates mit den Knochentrapekeln, wodurch für einen besonders festen Halt des Implantates im Knochen gesorgt wird.

Die Stielteile werden bei einer etwaigen notwendig werdenden Revision im Knochen belassen und nur die Verschleißteile wie das Aufnahmeteil und ggf. die Gelenkkugel werden ersetzt und das Gelenk daraufhin wieder zusammengesetzt.

Die Stabilität des Sitzes des ersten Stielteils im Trapezium wird noch dadurch erhöht, wenn vorteilhaft die dem Trapezium zuzuwendende Seite des ersten Stielteils zumindest teilweise mit einer offenmaschigen dreidimensionalen Raumnetzstruktur versehen ist. In diese Raumnetzstruktur hinein und durch diese hindurch können wiederum Knochentrapekel wachsen und so für eine stabile Langzeitfixation sorgen.

Es wird besonders bevorzugt, wenn an der Gelenkkugel ein konischer Verbindungszapfen angeformt ist, der mit einer im ersten Stielteil ausgeformten konischen Klemmhülse in einem konischen Klemmsitz sitzt. Im Revisionsfall kann die konische Verklemmung aufgehoben werden und die Gelenkkugel zusammen mit dem Zapfen ausgetauscht werden durch ein neues Teil.

Gemäß einer vorteilhaften Weiterbildung ist vorgesehen, dass das erste Stielteil auf seiner dem Trapezium zuzuwendenden Seite mit mindestens einem Antirotationsschild versehen ist. Dieser Antirotationsschild wird in das Trapezium eingetrieben und dient der Rotationssicherung.

Besonders bevorzugt besteht das Aufnahmeteil aus Polyethylen, und zwar hochverdichtetem Polyethylen. Es wird mit dem zweiten Stielteil in einer Art Rastverbindung verbunden, die jedoch unter Einsatz von entsprechenden Werkzeugen im Revisionsfall wieder gelöst werden kann, so dass auch dieses Teil leicht ausgetauscht werden kann unter Belassung der Stielteile in den jeweiligen Knochen.

Die Erfindung wird anhand eines Ausführungsbeispiels gemäß der Zeichnungsfiguren näher erläutert. Hierbei zeigt:
- Fig. 1: schematisch die Lage des implantierten Daumensattelgelenks in den Handknochen,
- Fig. 2: eine vergrößerte Ansicht des implantierten Daumensattelgelenks, und
- Fig. 3: in Explosionsdarstellung das Daumensattelgelenk in einer Ansicht (a), in einer gegenüber (a) um 90° verdrehten Lage (b) und eine schematische Schnittansicht.

Nachfolgend sind gleiche Teile mit denselben Bezugszeichen versehen.

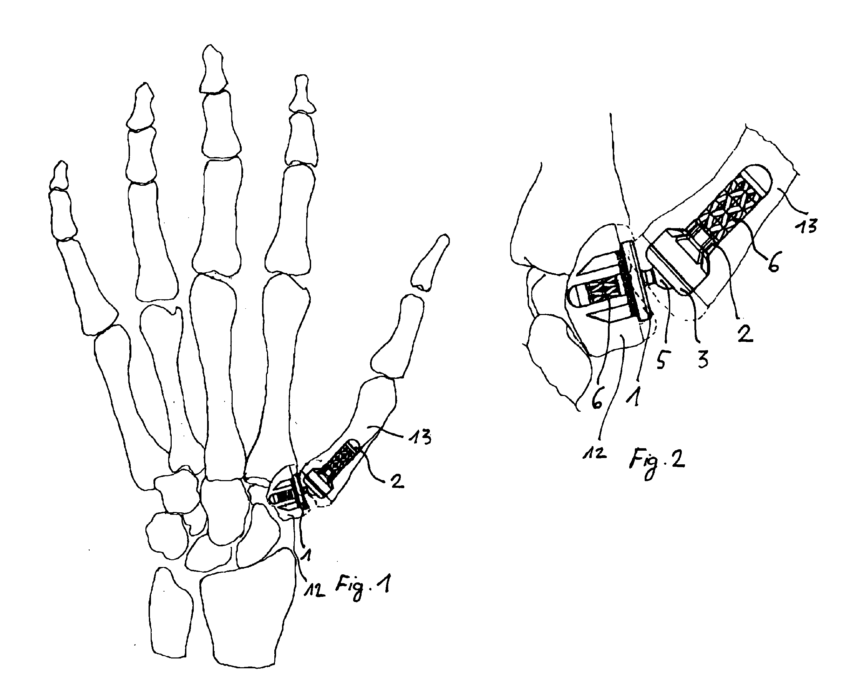

Einen ersten Überblick verschafft Fig. 1. Fig. 1 zeigt schematisch ein Handskelett.

Zwischen dem Trapezium 12 und dem Os metacarpale I 13 ist das Daumensattelgelenk implantiert. Dieses besteht im Wesentlichen aus einem ersten Stielteil 1, welches in das Trapezium 12 implantiert wurde, aus dem zweiten Stielteil 2, welches im Os metacarpale I 13 sitzt sowie aus einem Aufnahmeteil und einer Gelenkkugel, wie sich aus den nachfolgenden Betrachtungen ergibt.

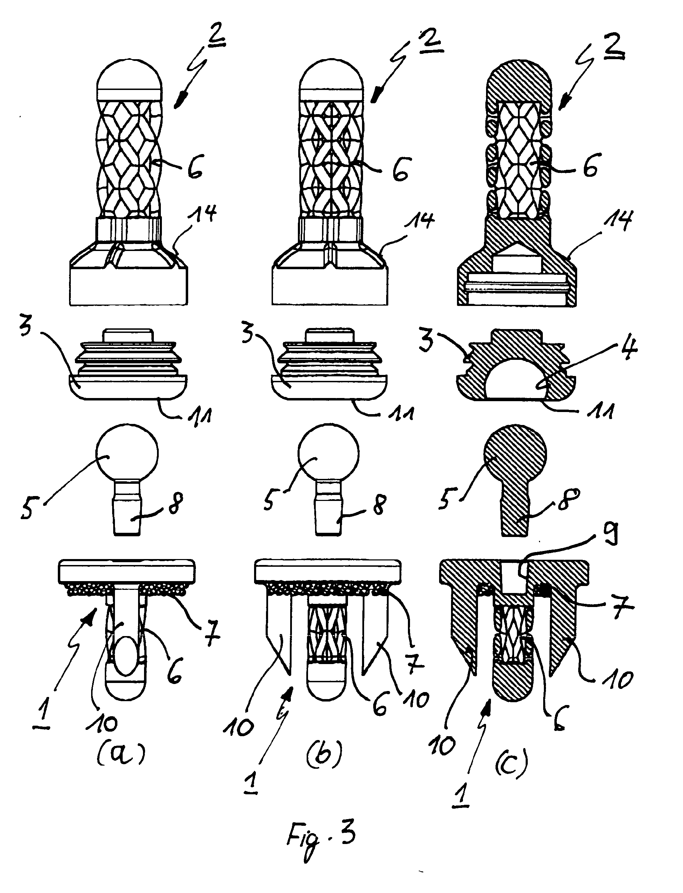

Hierzu wird auf Fig. 2 verwiesen. Zusätzlich zu den schon angesprochenen Teilen ist eine mit dem ersten Stielteil 1 lösbar verbundene Gelenkkugel 5 vorgesehen. Mit dem zweiten Stielteil 2 lösbar verbunden ist ein Aufnahmeteil 3. Die Gelenkkugel 5 liegt in einer kalottenförmigen Ausnehmung im Aufnahmeteil 3, die in Fig. 3(c) erkennbar ist.

Die Stielteile 1 und 2 sind vorliegend als Hohlstiele mit gitterstrukturierter Außenwandung 6 ausgebildet. Dies gestattet ein Ein- und Durchwachsen der Hohlstiel mit Knochenmaterial zu Langzeitfixation.

Weitere Einzelheiten sind den Darstellungen gemäß der Fig. 3(a) bis (c) zu entnehmen. Nachfolgend wird nur auf die Ausbildungen hingewiesen, die nicht schon Gegenstand der vorstehenden Beschreibung waren.

Die dem Trapezium 12 zuzuwendende Seite des ersten Stielteils 1 ist hier vorliegend vollständig mit einer offenmaschigen, dreidimensionalen Raumnetzstruktur 7 versehen, in die und durch die hindurch Knochentrapekel zur weiteren Förderung der Langzeitstabilität einwachsen.

Im dargestellten Ausführungsbeispiel ist das erste Stielteil 1 auf der dem Trapezium 12 zuzuwendenden Seite mit zwei Antirotationsschilden 10 versehen. Diese werden in das Trapezium 12 eingetrieben (Fig. 2), um so für eine Rotationssicherheit des Stielteils 1 im Trapezium 12 zu sorgen. Damit das Eintreiben der Rotationsschilde 10 möglichst mühelos vorgenommen werden kann, weisen die Rotationsschilde 10 eine messerartige Kontur auf (Fig. 3(b)).

In der schematischen Schnittansicht gemäß Fig. 3(c) ist die Ausnehmung 4 in dem Aufnahmeteil 3 deutlich erkennbar. Die Gelenkkugel 5 kommt in der Ausnehmung 4 zu liegen und rollt bei Ausübung der Gelenkfunktion in dem Aufnahmeteil 3 ab. Die Dimensionen sind so gewählt, dass die Basiskante 11 der Ausnehmung 4 über den Äquator der Gelenkkugel 6 greift, um insofern einen Luxationsschutz auszuüben.

Das Aufnahmeteil 3 wird mit einer Art Schnappverschluss mit dem komplementär ausgebildeten Kopf des zweiten Stielteils 2 so verbunden, dass es in einem Revisionsfall wieder entfernt und gegen ein neues Aufnahmeteil 3 ausgetauscht werden kann.

Die Austauschbarkeit der Gelenkkugel 5 ist gewährleistet durch einen angeformten konischen Verbindungszapfen 8, der mit einer im ersten Stielteil 1 ausgeformten konischen Klemmhülse 9 (Fig. 3(c)) in einem konischen Klemmsitz sitzt.

## Patentansprüche

1. Daumensattelgelenk, aufweisend
- ein in das Trapezium (12) implantierbares erstes Stielteil (1),
- ein in den Os metacarpale I (13) implantierbares zweites Stielteil (2),
- ein mit dem zweiten Stielteil (2) lösbar verbundenes Aufnahmeteil (3) mit einer kugelkalottenförmigen Ausnehmung (4), und
- eine mit dem ersten Stielteil (1) lösbar verbundene Gelenkkugel (5), die in der Ausnehmung (4) des Aufnahmeteils (3) liegt und bei Beugung des Gelenkes darin abrollt,
- wobei die Basiskante (11) der Ausnehmung (4) über den Äquator der Gelenkkugel (5) greift.

2. Daumensattelgelenk nach Anspruch 1, bei dem die Stielteile (1, 2) als Hohlstiele mit gitterstrukturierter Außenwandung (6) ausgebildet sind.

3. Daumensattelgelenk nach Anspruch 1 oder 2, bei dem die dem Trapezium (12) zuzuwendende Seite des ersten Stielteils (1) zumindest teilweise mit einer offenmaschigen dreidimensionalen Raumnetzstruktur (7) versehen ist.

4. Daumensattelgelenk nach einem der Ansprüche 1 bis 3, bei dem an der Gelenkkugel (5) ein konischer Verbindungszapfen (8) angeformt ist, der mit einer im ersten Stielteil (1) ausgeformten konischen Klemmhülse (9) in einem konischen Klemmsitz sitzt.

5. Daumensattelgelenk nach einem der Ansprüche 1 bis 4, bei dem das erste Stielteil (1) auf seiner dem Trapezium (12) zuzuwendenden Seite mit mindestens einem Antirotationsschild (10) versehen ist.

6. Daumensattelgelenk nach einem der Ansprüche 1 bis 5, bei dem das Aufnahmeteil (3) aus Polyethylen besteht.
